# EUROPEAN PATENT SPECIFICATION

(11) **EP 0 906 020 B1**
(45) Date of publication and mention of the grant of the patent: **06.02.2002**
(21) Application number: 97928177.1
(22) Date of filing: 09.06.1997
(51) Int. Cl.: A01N 63/00

(54) **USE OF A FUNGAL COMPOSITION FOR CROP PROTECTION**
VERWENDUNG EINER FUNGIZIDEN ZUSAMMENSETZUNG ALS PFLANZENSCHUTZMITTEL
UTILISATION D'UNE COMPOSITION FONGICIDE COMME AGENT PHYTOSANITAIRE

(30) Priority: 07.06.1996 EP 96201651
(43) Date of publication of application: 07.04.1999
(73) Proprietor: DSM N.V., 6411 TE Heerlen (NL)
(72) Inventor: BEUDEKER, Robert Franciscus, NL-2635 KC Den Hoorn (NL)
(74) Representative: Visser-Luirink, Gesina, Dr.
(86) International application number: EP9703046
(87) International publication number: WO9747202

(56) References cited:
- EP-A- 0 702 897
- FR-A- 2 385 730

## Description

The present invention relates to the use of compositions for combatting (or killing) yeasts and filamentous fungi.

Fungicides are often used in crop protection, disinfection, cleaning and even cosmetics and pharmaceuticals.

In many of these uses the fungicides place an undesirable burden on the environment. However, fungus related diseases result in reductions of crop yields and present a health hazard for animals and humans due to the production of mycotoxins which may enter the food chain.

The cell wall of fungi is composed of carbohydrates such as chitin, glucan and mannan. Chitin is a polysaccharide composed of N-acetyl-D-glucosamine linked by β(1>4)-glucosidic linkages. Mannans are composed of D-mannan, linked in the β-configuration by β(1>4)-,β(1>6)- and β(1>3)-linkages. β-glucans are homopolymers of D-glucose linked in the β-configuration. The occurrence and relative importance of these carbohydrates varies between classes of fungi. Oomycetes, for example, are characterized by a lack of chitin in the cell wall but do contain glucan and mannan.

Enzymes have been characterized which are capable of degrading fungal cell walls. These enzymes comprise endochitinases (EC 3.2.1.14), which randomly cleave chitin; chitobiosidases (chitin l,4-β-chitobiosidase; EC 3.2.1.30) which cleave dimeric units from one end of chitin; 1,3-β-glucanases (glucan-1,3-β-glucosidase; EC 3.2.1.58), which cleave 1,3-β-glucans; and glucosaminidase (N-acetyl-β-D-glucosaminidase; EC 3.2.1.30), which cleave monomeric units from one end of chitin and have N-acetyl-β-glucosaminidase activity.

Several classes of micro-organisms secrete these enzymes into their environment. In particular, enzymes produced by the fungus *Trichoderma* harzianum and *Gliocladum* virens have been studied in detail. Genes encoding these enzymes have been cloned and the antifungal activities of these enzymes have been tested against plant pathogenic fungi. A variety of synergistic antifungal effects between two or more single purified enzymes have been demonstrated (Lorito, M., Hayes, C.K., DiPietro, A., Woo, S.L. and Harman, G.E., Phytopathology 84, 398-405 (1994).

Combinations of chitinolytic and glucanolytic enzymes have been shown to be very effective in improving the fungicidal activity of the fungicides gliotoxin, flusilazole, miconazole, captan and benomyl thereby allowing a significant reduction in the chemical doses required (Lorito, M., Hayes, C.K., Zoina, A., Scala, F., Del Sorbo, G., Woo, S.L. and Harman, G.E., Molecular Biotechnology 2: 209-217 (1994)).

International Patent Application WO94/13784 describes combinations of fungal cell wall degrading enzymes and specific fungicides such as flusilazole, miconazole and captan.

The experiments described in the prior art have been conducted on a very small scale in the lab by using in *vitro* antifungal bioassays as described by Lorito, M., Harman, G.E., Hayes, C.K., Broadway, R.M., Tronsmo, A., Woo, S.L. and DiPietro, A., Phytopathology 83, 302-307 (1993). However, it is common that results in the lab cannot be applied to practical situations.

Moreover, the fungicides disclosed in the above mentioned patent application are all either sterol synthesis inhibitors or thiol group inactivators.

The present invention thus provides the use of a fungicidal composition comprising a polyene macrolide antibiotic together with at least one fungal cell wall degrading enzyme for treating the plant or a part there of.

Polyene macrolide antibiotics to be used according to the invention include compounds such as pimarycin (natamycin) and nystatin. They are rarely, if ever, used in crop protection because of their high price, but are used in, for example, pharmaceutical fungicidal compositions (See FR 2 385 730).

The combination of the macrolide and the cell wall degrading enzyme results in significant reduction in the amount of fungicide required so that the expensive macrolides can be applied as crop protection agents.

The polyene macrolide antifungal substances to be used according to the invention include, but are not limited to nystatin, natamycin, amphotericin B, candicidin, filipin, homycin, etruscomycin and trichomycin. Some of these macrolide compounds are mixtures of different active substances. These polyene macrolide antibiotics are characterized by a macrolide ring. They differ in the number (12-37) of carbon atoms in the ring structure, the number of hydroxyl groups (6-14) and the presence or absence of a carbohydrate. Polyene macrolide antibiotics alter the membrane permeability of fungal cells by forming a complex with sterol. As a result a fatal loss of potassium occurs.

The preferred fungicides to be used according to the invention are natamycin and/or nystatin. These are very effective fungicides which show synergistic antifungal action in combination with cell wall degrading enzymes, particularly with β-1,3-glucanase, which is therefore a preferred cell wall degrading enzyme for use in the compositions according to the invention. However, β-1,3-glucanase may also break down plant cell walls and therefore the amount of this enzyme should be limited to 500,000 U/l (or kg), preferably 50,000 U/l (or kg), more preferably 10,000 U/l (or kg). Its effectiveness, even in small amounts, can be maintained by using a combination of different cell wall degrading enzymes, such as a combination of β-1,3-glucanase and an enzyme which breaks down the components of fungal cell walls not present in plant cell walls. Such enzymes are for instance chitinase or mannanase. However, other enzymes may also be used, either alone or in combination. Useful enzymes, some of which have already been mentioned, include but are not limited to: cellulases, in particular exo/endoglucanases, such as β-1,3-glucanase or β-1,4-glucanase; exo/endochitinases; mannanases; galactanases and proteases.

The enzymes may be obtained from any organism which produces them. The exemplified enzymes have been obtained from *Trichoderma longibrachiatum*, but other micro-organisms are a source of enzymes as are plant cells, yeast cells, fungi and even animal or insect cells. It is of course clear that genes encoding cell wall degrading enzymes may be incorporated into any suitable host cell to facilitate production of the enzymes. Many useful enzymes have been disclosed on pages 4-12 of WO94/13784 which is incorporated herein by reference.

The nature of the antifungal compositions is determined by the use and is dependent on, for example, the manner of application and the effective dose.

Preferred areas of application include but are not limited to: antifungal treatment of seeds, bulbs, fruits, plants, silage, food, feed or fodder . Calculation of the required dosages may be performed by any person skilled in the art.

Compositions used according to the invention will typically contain between 1 and 1000 mg/l of fungicide and between 50 and 500,000 Units of each enzyme/l (or kg), preferably between 1 and 500 mg/l fungicide and between 50 and 50,000 Units of each enzyme/l (or kg), and most preferably between 1 and 250 mg/l fungicide and between 50 and 10,000 Units of each enzyme/l (or kg).

The compositions used according to the invention can be used in essentially the same way as prior art antifungal compositions.

The compositions can be typically applied to seeds, roots, foliage or fruit. The preferred agricultural products to be treated with the compositions according to the invention are flower bulbs.

For flower bulbs it is common to treat them with hot-water prior to planting to control parasitic insects, mites and nematodes. Such a treatment may prevent the spread of pathogenic micro-organisms (Langerak, 1985; PhD thesis entitled "The pathogenesis of *Fusarium oxysporium f.sp. nacissi* and the role of antagonistic bulb-borne fungi in the chemical control of basal rot" Agricultural University Wageningen, The Netherlands).

Antimicrobial agents are added to the hot-water bath to reduce the spread of micro-organisms. The potential use of the antifungal agent natamycin (a polyene macrolide produced by *Streptomyces natalensis*) in this application has been demonstrated in the past (Langerak supra). Practical applications before now have been very limited due to the fact that other antifungal agents appeared to show a superior price: performance ratio. However, the effective dose rate of natamycin (or other fungicidal polyene macrolides) may be lowered considerably if sufficient units of the fungal cell wall degrading enzymes chitinase and β-1,3-glucanase are added according to the present invention. This finding improves the price performance ratio of natamycin considerably and enables commercial application.

In addition, mixing of fungicidal polyene macrolides such as natamycin and fungal cell wall degrading enzymes in the soil is a very effective way to control infection of flower bulbs by *Rhizoctonia*. Synergistic effects of fungicidal polyene macrolides such as natamycin and fungal cell wall degrading enzymes such as chitinase and β-1,3-glucanase result in the decrease of the necessary dose and can improve the price: performance ratio significantly.

A composition used according to the invention can thus be added to the said hot-water bath, or the composition may be used as the hot aqueous bath itself.

The following examples are designed to illustrate and in no way limit the scope of the present invention.

### Example 1

### Production of fungal cell wall degrading enzymes

Enzymes were derived from a commercial fermentation of the fungus *Trichoderma longibrachiatum*. Broth of *T. longibrachiatum* was subjected to plate filtration followed by ultrafiltration. The ultrafiltrate was then subjected to fluid bed granulation according to procedures known to persons skilled in the art. Sodium sulphate was used as a nucleus during fluid bed granulation.

The tested preparation contained 180 units of endochitinase activity per gram of granulated product. One unit is defined as the amount of enzyme that liberates 1 mg N-acetyl-D-glucosamine from cm-chitin-rbv (Sigma catalogue number C 3020) at pH 6.0 at 25°C per 48 hours.

The method has been described in detail in Analytical Biochemistry 8: 397-401 (1964). The same preparation also contained 50 units of β-1,3-glucanase per gram of granulated product. One unit is defined as the amount of enzyme that liberates 1 micromole of reducing sugars per minute from 0.1% (w/v) laminarin (Sigma catalogue number L9634) at pH 6.7 at 30°C. This method has been described in detail in Molecular Biotechnology 2: 209-217 (1994).

### Example 2

### Synergistic effects of fungal cell wall degrading enzymes and natamycin against Fusarium in flower bulbs

Viability of conidia of *Fusarium oxysporum* was measured at the start and at the end of the hot-water treatment. (2 hrs at 43.5°C).
When no fungicidal agents were added, duplicate samples of 5 ml were taken from the water in the bath at the beginning and the end of the treatment, and diluted with sterile water so that 1 ml did not contain more than 200 living conidia. Five samples of 1 ml were mixed each with 20 ml of PDA containing 50 microgram/ml vendarcin (PDA-V) and poured into petri dishes of 14 cm diameter. Colonies were counted after 2 and 5 days at 25°C.

In the presence of natamycin and fungal cell wall degrading enzymes, 2 samples of 25 ml were taken from the bath at the beginning and the end of the treatment. These samples were centrifuged at 3,000 * g for 15 min. The pellet containing the conidia was washed several times with sterile water to remove the antifungal agents, followed by centrifugation and dilution to allow for plating on PDA-V as described above.

Natamycin was added to the hot-water bath to a final concentration of 300 mg/L in the absence of fungal cell wall degrading enzymes. A series of natamycin concentrations were tested to demonstrate synergy with chitinase and β-1,3-glucanase.

Chitinase and β-1,3-glucanase respectively were added to the hot-water bath to a final activity of 36,000 and 10,000 units per liter. Units are as defined in Example 1. Results are shown in Table 1.

**Table 1**

| Effects on survival of hot-water treated conidia (25,000 conidia per ml) of *Fusarium oxysporum* as measured on PDA-V agar. Fungal cell wall degrading enzymes and different concentrations of natamycin were added to the hot-water bath | |
|---|---|
| **Treatment** | **Surviving conidia per ml** |
| No addition | 100 |
| Natamycin 300 mg/L | <1 |
| Natamycin 200 mg/L | 50 |
| Natamycin 100 mg/L | 70 |
| Natamycin 100 mg/L plus fungal cell wall degrading enzymes | <1 |

### Example 3

### Synergistic effects of fungal cell wall degrading enzymes and natamycin in the soil against Rhizoctonia in flower bulbs

Bulbs were planted in pots at standard soil at a depth of 20 cm. Oat borne sclerotia of *Rhizoctonia* were mixed through the soil to infect the bulbs. Temperature was maintained at 18°C during the experiment. Experiments were replicated 5 fold.

Natamycin and fungal cell wall degrading enzymes were mixed as powders through the soil prior to infection with *Rhizoctonia.*

Natamycin was tested at final concentrations of 1000, 500 and 100 mg/kg. Chitinase and β-1,3-glucanase was mixed through the soil to a final activity of 3600 and 1000 units per kg of soil.

After 4 weeks bulbs were monitored for fungal infection by means of visual inspection. Results are shown in Table 2.

**Table 2**

| **Treatment** | **Percentage of bulbs showing fungal infection** |
|---|---|
| No treatment | 61 |
| Natamycin 1000 mg/kg | 19 |
| Natamycin 500 mg/kg | 42 |
| Natamycin 100 mg/kg | 50 |
| Natamycin 100 mg/kg plus fungal cell wall degrading enzymes | 21 |

The results demonstrate the synergistic effects of natamycin and fungal cell wall degrading enzymes on the plant pathogenic fungus *Rhizoctonia*. Differences between the various treatments are statistically significant except for the groups "no treatment" and natamycin at a dose of 100 mg/kg.

## Claims

1. Use of a fungicidal composition comprising a polyene macrolide antibiotic and at least one fungal cell wall degrading enzyme in the antifungal treatment of seeds, bulbs, fruits, plants, silage, food, feed or fodder.

2. Use according to claim 1, wherein the polyene antibiotic is natamycin or nystatin.

3. Use according to claim 1 or 2, wherein the cell wall degrading enzyme comprises a cellulase, a glucanase, a mannanase, a chitinase, a galactanase or a protease.

4. Use according to anyone of claims 1 to 3, wherein the cell wall degrading enzyme is a β-1,3-glucanase, a chitinase and/or a mannanase.

5. Use according to claim 4, wherein the the cell wall degrading enzyme consists of a β-1,3-glucanase together with a chitinase or a mannanase.

6. Use according to any one of claims 1 to 5, wherein the amount of antibiotic used ranges from 1 to 1000 mg/l, preferably from 1 to 500 mg/l and more preferably from 1 to 250 mg/l.

7. Use according to any one of claims 1 to 6, wherein the amount of each enzyme used ranges from 50 to 500,000 Units/I, preferably from 50 to 50,000 Units/l, more preferably from 50 to 10,000 Units/I.

8. Use according to any one of claim 1 to 7, wherein the treatment is of seed potatoes.

9. A method of treating a plant or part thereof, the method comprising contacting the plant or part thereof with a fungicidal composition as defined in any one of the claims 1 to 7.

10. A method according to claim 9, wherein the part of a plant is a seed, tuber, bulb or fruit.

11. A part of a plant treated by a method according to claim 9 or 10.

## Patentansprüche

1. Verwendung eines fungiziden Gemisches, das ein Polyenmakrolid-Antibiotikum und mindestens ein Pilzzellwände abbauendes Enzym enthält, bei der Antipilzbehandlung von Samen, Zwiebeln, Früchten, Pflanzen, im Silo konserviertem Grünfutter, Nahrungsmitteln, Tierfutter oder Trokkenfutter.

2. Verwendung nach Anspruch 1, worin das Polyen-Antibiotikum Natamycin oder Nystatin ist.

3. Verwendung nach Anspruch 1 oder 2, worin das Zellwände abbauende Enzym eine Cellulase, eine Glucanase, eine Mannanase, eine Chitinase, eine Galactanase oder eine Protease enthält.

4. Verwendung nach einem der Ansprüche 1 bis 3, worin das Zellwände abbauende Enzym eine β-1,3-Glucanase, eine Chitinase und/oder eine Mannanase ist.

5. Verwendung nach Anspruch 4, worin das Zellwände abbauende Enzym aus einer β-1,3-Glucanase zusammen mit einer Chitinase oder einer Mannanase besteht.

6. Verwendung nach einem der Ansprüche 1 bis 5, worin die Menge des eingesetzten Antibiotikums im Bereich von 1 bis 1000 mg/l, vorzugsweise von 1 bis 500 mg/l und insbesondere von 1 bis 250 mg/l, liegt.

7. Verwendung nach einem der Ansprüche 1 bis 6, worin die Menge jedes Enzyms im Bereich von 50 bis 500.000 Einheiten/l, vorzugsweise von 50 bis 50.000 Einheiten/l, insbesondere von 50 bis 10.000 Einheiten/l, liegt.

8. Verwendung nach einem der Ansprüche 1 bis 7, worin die Behandlung auf Kartoffelsamen gerichtet ist.

9. Verfahren zum Behandeln einer Pflanze oder eines Teils hiervon, wobei das Verfahren das Inkontaktbringen der Pflanze oder eines Teils hiervon mit einem fungiziden Gemisch, wie es in einem der Ansprüche 1 bis 7 definiert ist, beinhaltet.

10. Verfahren nach Anspruch 9, worin der Teil der Pflanze ein Samen, eine Knolle, eine Zwiebel oder eine Frucht ist.

11. Teil einer Pflanze, der nach einem Verfahren gemäß Anspruch 9 oder 10 behandelt worden ist.

## Revendications

1. Utilisation d'une composition fongicide comprenant un antibiotique macrolide polyénique et au moins une enzyme apte à dégrader la paroi de cellules fongiques dans le traitement antifongique de graines, bulbes, fruits, plantes, ensilage, aliments, aliments pour animaux ou fourrage.

2. Utilisation selon la revendication 1, dans laquelle l'antibiotique polyénique est la natamycine ou la nystatine.

3. Utilisation selon la revendication 1 ou 2, dans laquelle l'enzyme apte à dégrader la paroi cellulaire comprend une cellulase, une glucanase, une mannanase, une chitinase, une galactanase ou une protéase.

4. Utilisation selon l'une quelconque des revendications 1 à 3, dans laquelle l'enzyme apte à dégrader la paroi cellulaire est une β-1,3-glucanase, une chitinase et/ou une mannanase.

5. Utilisation selon la revendication 4, dans laquelle l'enzyme apte à dégrader la paroi cellulaire comprend une β-1,3-glucanase conjointement avec une chitinase ou une mannanase.

6. Utilisation selon l'une quelconque des revendications 1 à 5, dans laquelle la quantité d'antibiotique utilisé est comprise entre 1 et 1000 mg/l, de préférence entre 1 et 500 mg/l et, de manière plus préférentielle, entre 1 et 250 mg/l.

7. Utilisation selon l'une quelconque des revendications 1 à 6, dans laquelle la quantité de chaque enzyme utilisée est comprise entre 50 et 500.000 unités/l, de préférence entre 50 et 50.000 unités/l et, de manière plus préférentielle, entre 50 et 10.000 unités/l.

8. Utilisation selon l'une quelconque des revendications 1 à 7, dans laquelle le traitement concerne les pommes de terre de semence.

9. Procédé permettant de traiter une plante ou une partie de celle-ci, le procédé comprenant la mise en contact de la plante ou d'une partie de celle-ci avec une composition fongicide telle que définie dans l'une quelconque des revendications 1 à 7.

10. Procédé selon la revendication 9, dans lequel la partie de plante est une graine, un tubercule, un bulbe ou un fruit.

11. Partie de plante traitée à l'aide du procédé selon la revendication 9 ou 10.
